# FASCICULE DE BREVET EUROPEEN

(11) **EP 1 647 257 B1**
(45) Date de publication et mention de la délivrance du brevet: **03.10.2012**
(21) Numéro de dépôt: 05300813.2
(22) Date de dépôt: 13.10.2005
(51) Int. Cl.: A61K 8/39, A61K 8/81, A61Q 1/10, A61K 8/04, A61K 8/87, A61K 8/86

(54) **Composition cosmétique de soin ou de maquillage, résistante à l'eau et facilement démaquillable, comprenant au moins un latex ou un pseudo-latex**
Wasserbeständige, einfach abschminkbare Schmink- oder Körperpflegezusammensetzung enthaltend einen Latex oder Pseudo-Latex
Water resistent, easy removable, make-up or personal care cosmetic composition comprising a latex or pseudo-latex

(30) Priorité: 13.10.2004 FR 0410824
(43) Date de publication de la demande: 19.04.2006
(73) Titulaire: L'Oréal, 75008 Paris (FR)
(72) Inventeur: PAYS, Karl, 94410, SAINT-MAURICE (FR); Bichon, Yohann, 94700, Maisons-Alfort (FR); Olivier-Mabilais, Sandrine, 94240, L' Hay les Roses (FR)
(74) Mandataire: Le Coupanec, Pascale A.M.P.

(56) Documents cités:
- EP-A- 0 628 304
- WO-A-01/01935
- WO-A-01/01936
- WO-A-02/47622
- FR-A- 2 844 196
- FR-A- 2 859 101

## Description

La présente invention concerne des compositions cosmétiques pour le maquillage et/ou le soin des matières kératiniques notamment de la peau, des lèvres et/ou des fibres kératiniques, susceptibles de résister à l'eau grâce à la présence d'au moins un latex ou pseudo-latex et d'être néanmoins facilement démaquillées.

Plus particulièrement, les compositions selon l'invention peuvent constituer un produit de maquillage du visage, du corps et/ou des lèvres et des fibres kératiniques, comme les cils, les sourcils et les cheveux, et plus particulièrement un produit de maquillage des cils.

Il peut notamment s'agir d'une composition de maquillage, d'une composition transparente ou colorée à appliquer sur ou sous un maquillage, dites encore respectivement "top-coat" ou "base-coat", ou bien encore d'une composition de traitement des cils.

La composition selon l'invention peut se présenter sous la forme d'un produit pour les cils ou mascara, d'un produit pour les sourcils, ou d'un produit de maquillage des cheveux. Plus spécialement, l'invention porte sur un mascara.

Il existe en pratique essentiellement deux types de formulation de mascara, à savoir d'une part, des mascaras à phase continue aqueuse, dits « mascaras émulsion », se présentant sous forme d'émulsion de cires dans l'eau et, d'autre part, des mascaras à phase continue solvant ou huile, anhydres ou à faible teneur en eau et/ou solvants hydrosolubles, dits « mascaras waterproof », formulés à l'état de dispersion de cires dans des solvants non aqueux. Il existe encore certains mascaras se présentant sous forme d'émulsions de cires dans l'eau, également qualifiés de "waterproof". Ces dernières compositions se caractérisent par la présence d'au moins un latex ou d'un pseudo-latex, à savoir d'une suspension colloïdale d'un polymère filmogène, qui confère la résistance à l'eau.

La présente invention est relative plus particulièrement aux compositions ou mascaras de type "waterproof" comprenant au moins un latex ou un pseudo-latex.

Ces mascaras dits "waterproof" comprenant au moins un latex ou un pseudo-latex peuvent donner lieu à un démaquillage difficile avec certains démaquillants, en particulier les démaquillants principalement aqueux ou hydrosolubles, notamment les solutions aqueuses. Le démaquillage s'effectue donc en règle générale à l'aide de démaquillants spécifiques à base d'huiles ou de solvants organiques. Or ces démaquillants peuvent être irritants pour l'oeil, notamment provoquer des picotements ou laisser un voile sur l'oeil, ou bien encore peuvent laisser sur la peau autour de l'oeil (paupières) un film résiduel gras inconfortable.

Il existe donc un besoin de disposer de compositions cosmétiques pour le maquillage susceptibles à la fois de présenter une bonne tenue à l'eau et à la fois une aptitude améliorée au démaquillage, y compris avec les démaquillants usuels.

Les documents FR 2 785 801, EP 1 152 022, FR 2 774 996, WO 95/35089 et WO 99/26445 décrivent des compositions épaississantes, dites « latex épaississant » ou « agent épaississant » ou encore « latex inverse ».

Les documents FR 2 785 801 et FR 2 774 996 divulguent notamment des compositions comprenant une phase aqueuse, une phase huileuse, un agent émulsifiant de type H/E (huile dans eau) et un agent émulsifiant de type E/H (eau dans huile) ainsi qu'un polyélectolyte anionique, branché ou réticulé, à base d'un monomère possédant une fonction acide fort.

Le document WO 99/52499 décrit principalement des rouges à lèvres comprenant un polyacrylate de sodium dans le but de produire un effet volumateur.

On a maintenant découvert qu'il était possible d'obtenir des compositions cosmétiques pour le maquillage et/ou le soin des matières kératiniques comprenant au moins un latex ou un pseudo-latex et capables de concilier une bonne tenue à l'eau et un démaquillage amélioré quel que soit le type de démaquillant.

La présente invention concerne une composition pour le maquillage et/ou le soin des matières kératiniques, ladite composition ayant une phase continue aqueuse et comprenant au moins une dispersion aqueuse de particules d'un polymère, au moins un polyélectrolyte en une teneur en matière sèche supérieure à 1 % en poids par rapport au poids total de la composition, et au moins un agent tensioactif de HLB supérieur ou égal à 6, en une teneur comprise entre 0,1% et 10% en poids par rapport au poids total de la composition ledit polymère étant présent en une teneur en matière sèche supérieure ou égale à 5 % en poids par rapport au poids total poids total de la composition, et ledit polyélectrolyte étant un copolymère acrylamide/acide 2-méthyl 2-[(1-oxo2propényl)amino] propene sulfonique.

Selon un deuxième aspect, la présente invention concerne un procédé cosmétique de maquillage et/ou de soin des matières kératiniques comprenant au moins une étape d'application sur les matières kératiniques de l'une quelconque des compositions telles que définies précédemment.

Selon un troisième aspect, la présente invention concerne l'utilisation selon la revendication 22 de l'association d'au moins un polyélectrolyte et d'au moins un agent tensioactif de HLB supérieur ou égal à 6 à 25°C, à titre d'additif pour conférer conjointement une bonne tenue à l'eau et une facilité de démaquillage à une composition cosmétique à phase continue aqueuse comprenant au moins une dispersion aqueuse de particules d'un polymère.

Comme il ressort des exemples ci-après, les compositions selon l'invention manifestent les avantages techniques exposés précédemment.

Par « composition cosmétique à phase continue aqueuse », on entend un système apte à se diluer ou se disperser au contact de l'eau.

Par "huile ou solvant organique volatile", on entend une huile ou solvant organique (ou milieu non aqueux) susceptible de s'évaporer au contact de la peau en moins d'une heure, à température ambiante et pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm de Hg), et de préférence allant de 1,3 Pa à 8000 Pa (0,01 à 60 mm de Hg).

Dans le cadre de la présente invention, "matières kératiniques" comprend la peau, les lèvres, les ongles, les cheveux, les cils et les sourcils.

Dans le cadre de la présente invention, par « fibres kératiniques » on entend notamment les cheveux, les cils et les sourcils. De plus, le maquillage de la peau s'entend notamment du maquillage du corps, des mains, du cou ou du visage.

La composition selon l'invention comprend un milieu physiologiquement acceptable, notamment cosmétiquement acceptable, c'est-à-dire un milieu compatible en particulier avec les matières kératiniques et notamment les fibres kératiniques telles que les cheveux, les cils, les sourcils.

Par "cosmétiquement acceptable", on entend, dans le cadre de la présente invention, un composé dont l'utilisation est compatible avec une application sur les matières kératiniques.

D'une manière générale, dans un souci de simplification, et sauf indication contraire, les teneurs sont indiquées en matière sèche.

### POLYELECTROLYTE

Par "polyélectrolyte", on entend une substance macromoléculaire, qui a la faculté de se dissocier, lorsqu'elle est dissoute dans l'eau ou dans tout autre milieu ionisant, pour donner au moins un ion. Autrement dit, un polyélectrolyte est un polymère comportant au moins un monomère ionisable.

En particulier, le polyélectrolyte peut donner des polyions, par exemple des polyanions, lorsqu'il est dissocié dans l'eau. Un polyélectrolyte peut être un polyacide, une polybase, un polysel ou polyampholyte. Dans le cadre de l'invention, il est de préférence un polyacide, et de préférence un polyacide fort.

De façon préférée, le polyélectrolyte compris dans les compositions cosmétiques selon la présente invention est un polymère anionique branché et/ou réticulé.

De préférence, le polyélectrolyte est en outre apte à former un gel en solution à 0,5 % en poids (en matière sèche).

Avantageusement, il se distingue également du polymère en dispersion aqueuse.

Les contre-ions des polyions formés lors de la dissociation peuvent être de toute nature, inorganique ou organique.

En particulier, lorsque le polyélectrolyte est un polymère anionique branché ou réticulé, les cations peuvent être des cations alcalins ou alcalino-terreux tels que le sodium ou le potassium ou encore l'ion ammonium.

Le cation sodium Na⁺ est préféré, c'est pourquoi il est principalement cité dans la liste de polyélectrolytes qui va suivre, sans que cela constitue une quelconque limitation à ce contre-ion spécifique.

A titre de polyélectrolyte, on peut citer:
- le copolymère acrylamide / AMPS de Na tel que le Simulgel 600^{®} sous forme d'émulsion contenant du polysorbate 80 à titre de tensioactif et contenant de l'isohexadécane à titre de phase huile, vendu par la société SEPPIC, ou encore le Simulgel EG^{®}, le Simulgel A^{®} et le Simulgel 501^{®} vendus par la même société.

Le Simulgel 600^{®} est notamment décrit dans le document FR 2 785 801. Il s'agit en réalité d'un latex inverse. Le polyélectrolyte AMPS est de l'acide 2-méthyl 2-[(1-oxo 2-propényl)amino] 1-propanesulfonique partiellement ou totalement salifié notamment sous forme de sel de sodium ou de sel d'ammonium compris à hauteur de 30 à 50 % en proportions molaires dans le mélange comprenant de l'AMPS ainsi qu'un acrylamide, contenu quant à lui à hauteur de 50 à 70 %.

Il sera bien entendu fait en sorte que la teneur en polyélectrolyte soit ajustée de telle manière que l'aptitude au démaquillage soit effectivement améliorée tout en étant non préjudiciable à la tenue à l'eau de la composition cosmétique.

Il est entendu que la quantité en polyélectrolyte est susceptible de varier significativement selon la nature du polyélectrolyte. De manière générale, cette quantité est au moins égale à la quantité nécessaire et suffisante pour conférer à ladite composition une meilleure aptitude au démaquillage. Elle est encore qualifiée de quantité efficace.

Cette aptitude au démaquillage peut notamment être appréciée à l'aide du test figurant dans les exemples ci-après.

Sans que cela constitue une quelconque limitation à l'invention, les inventeurs ont émis l'hypothèse que le polyélectrolyte joue le rôle de « pompe à eau ». Ainsi, ce rôle de « pompe à eau » apparaît plus nettement lorsque la composition est mise en contact avec une phase aqueuse au moment du démaquillage. Lors du démaquillage, le film de la composition cosmétique selon l'invention se fragilise en surface et entraîne sa rupture mécanique ; Il y a alors morcellement du film.

Le polyélectrolyte peut aussi être présent dans la composition dans une teneur allant de 1 à 15 % en poids, en particulier de 1,5 à 10 % en poids, et notamment de 2 à 7 % en poids par rapport au poids total de la composition.

### TENSIOACTIF

Les compositions selon l'invention contiennent au moins un agent tensioactif de HLB supérieur ou égal à 6 à 25 °C.

Il est entendu que lorsque le polyélectrolyte est incorporé à la composition de l'invention sous forme d'une composition déjà formulée avec un agent tensioactif de HLB supérieur ou égal à 6 à 25 °C, la quantité en agent tensioactif ci-dessus définie tient compte de la teneur en ledit agent tensioactif comprise dans la formulation du polyélectrolyte.

Le « HLB supérieur ou égal à 6 » s'entend d'un tensioactif possédant à 25 °C une balance HLB (hydrophile-lipophile-balance) au sens de GRIFFIN supérieure ou égale à 6.

La valeur HLB selon GRIFFIN est définie dans J. Soc. Cosm. Chem. 1954 (volume 5), pages 249-256.

On peut se reporter au document « Encyclopedia of Chemical Technology, KIRK-OTHMER », volume 22, p. 333-432, 3ème édition, 1979, WILEY, pour la définition des propriétés et des fonctions (émulsionnant) des tensioactifs, en particulier p. 347-377 de cette référence, pour les tensioactifs non ioniques.

L'agent tensioactif de HLB supérieur ou égal à 6 à 25 °C compris dans la composition cosmétique selon la présente invention peut être ionique, non ionique ou à caractère mixte ionique et non ionique.

Conviennent tout particulièrement à la présente invention, les compositions cosmétiques où les tensioactifs de HLB supérieur ou égal à 6 à 25 °C sont non ioniques.

Parmi les agents tensioactifs non ioniques de HLB supérieur ou égal à 6 à 25 °C pouvant être présents dans les compositions selon la présente invention, utilisés seuls ou en mélange, on peut citer notamment :
- les éthers oxyéthylénés et/ou oxypropylénés (pouvant comporter de 1 à 150 groupes oxyéthylénés et/ou oxypropylénés) de glycérol ;
- les éthers oxyéthylénés et/ou oxypropylénés (pouvant comporter de 1 à 150 groupes oxyéthylénés et/ou oxypropylénés) d'alcools gras (notamment d'alcool en C₈-C₂₄, et de préférence en C₁₂-C₁₈) tels que l'éther oxyéthyléné de l'alcool cétéarylique à 30 groupes oxyéthylénés (nom CTFA "Ceteareth-30 ") et l'éther oxyéthyléné du mélange d'alcools gras en C₁₂-C₁₅ comportant 7 groupes oxyéthylénés (nom CTFA "C₁₂-C₁₅ Pareth-7" commercialisé sous la dénomination NEODOL 25-7® par SHELL CHEMICALS,
- les esters d'acide gras (notamment d'acide en C₈-C₂₄, et de préférence en C₁₆-C₂₂) et de polyéthylène glycol (pouvant comprendre de 1 à 150 motifs d'éthylèneglycol) tels que le stéarate de PEG-50 et le monostéarate de PEG-40 commercialisé sous le nom MYRJ 52P® par la société ICI UNIQUEMA,
- les esters d'acide gras (notamment d'acide en C₈-C₂₄, et de préférence en C₁₆-C₂₂) et des éthers de glycérol oxyéthylénés et/ou oxypropylénés (pouvant comporter de 1 à 150 groupes oxyéthylénés et/ou oxypropylénés), comme le monostéarate de PEG-200 glycéryle vendu sous la dénomination Simulsol 220 TM^{®} par la société SEPPIC ; le stéarate de glycéryle polyéthoxylé à 30 groupes d'oxyde d'éthylène comme le produit TAGAT S^{®} vendu par la société GOLDSCHMIDT, l'oléate de glycéryle polyéthoxylé à 30 groupes d'oxyde d'éthylène comme le produit TAGAT O^{®} vendu par la société GOLDSCHMIDT, le cocoate de glycéryle polyéthoxylé à 30 groupes d'oxyde d'éthylène comme le produit VARIONIC LI 13^{®} vendu par la société SHEREX, l'isostéarate de glycéryle polyéthoxylé à 30 groupes d'oxyde d'éthylène comme le produit TAGAT L^{®} vendu par la société GOLDSCHMIDT et le laurate de glycéryle polyéthoxylé à 30 groupes d'oxyde d'éthylène comme le produit TAGAT I^{®} de la société GOLDSCHMIDT,
- les esters d'acide gras (notamment d'acide en C₈-C₂₄, et de préférence en C₁₆-C₂₂) et des éthers de sorbitol oxyéthylénés et/ou oxypropylénés (pouvant comporter de 1 à 150 groupes oxyéthylénés et/ou oxypropylénés), comme le polysorbate 60 vendu sous la dénomination Tween 60^{®} par la société UNIQUEMA, ainsi que le polysorbate 80, le polysorbate 40 et le polysorbate 20,
- la diméthicone copolyol, telle que celle vendue sous la dénomination Q2-5220^{®} par la société DOW CORNING,
- la diméthicone copolyol benzoate (FINSOLV SLB 101^{®} et 201^{®} de la société FINTEX),
- les copolymères d'oxyde propylène et d'oxyde d'éthylène, également appelés polycondensats OE/OP,
- et leurs mélanges.

Les polycondensats OE/OP sont plus particulièrement des copolymères consistant en des blocs polyéthylène glycol et polypropylène glycol, comme par exemple les polycondensats tribloc polyéthylène glycol/polypropylène glycol/polyéthylène glycol. Ces polycondensats tribloc ont par exemple la structure chimique suivante :

H-(O-CH₂-CH₂)ₐ-(O-CH(CH₃)-CH₂)_{b}-(O-CH₂-CH₂)ₐ-OH,

formule dans laquelle a va de 2 à 120, et b va de 1 à 100.

Le polycondensat OE/OP a de préférence un poids moléculaire moyen en poids allant de 1000 à 15000, et de mieux allant de 2000 à 13000. Avantageusement, ledit polycondensat OE/OP a une température de trouble, à 10 g/l en eau distillée, supérieure ou égale à 20 °C, de préférence supérieure ou égale à 60 °C. La température de trouble est mesurée selon la norme ISO 1065. Comme polycondensat OE/OP utilisable selon l'invention, on peut citer les polycondensats tribloc polyéthylène glycol / polypropylène glycol / polyéthylène glycol vendus sous les dénominations SYNPERONIC^{®} comme les SYNPERONIC PE/ L44^{®} et SYNPERONIC PE/F127^{®} par la société ICI.

Parmi les agents tensioactifs ioniques, qui peuvent être anioniques ou cationiques, de HLB supérieur ou égal à 6 à 25 °C pouvant être présents dans la composition selon la présente invention, utilisés seuls ou en mélange, on peut citer notamment :
- les tensioactifs siliconés comme les diméthicones copolyols phosphates tels que celui vendu sous la dénomination PECOSIL PS 100^{®} par la société PHOENIX CHEMICAL,
- les dérivés d'acides aminés, tels que le lauryl sarcosinate, et le lauryl taurate,
- les sels d'acides gras en C₁₆-C₃₀, notamment ceux dérivant des amines, comme le stéarate de triéthanolamine,
- les sels d'acides gras polyoxyéthylénés, notamment ceux dérivant des amines ou les sels alcalins, et leurs mélanges,
- les esters phosphoriques et leurs sels tels que le «DEA oleth-10 phosphate » (Crodafos N 10N de la société CRODA),
- les sulfosuccinates tels que le «Disodium PEG-5 citrate lauryl sulfosuccinate » et le « Disodium ricinoleamido MEA sulfosuccinate »,
- les alkyléthersulfates tels que le lauryl éther sulfate de sodium,
- les iséthionates,
- les acylglutamates tels que le « Disodium hydrogenated tallow glutamate » (AMISOFT HS-21 R commercialisé par la société AJINOMOTO) et leurs mélanges.

Convient tout particulièrement à l'invention le stéarate de triéthanolamine. Ce dernier est généralement obtenu par simple mélange de l'acide stéarique et de la triéthanolmaine.

A titre représentatif des tensioactifs cationiques, on peut notamment citer :
- les alkyl-imidazolidinium tels que l'étho-sulfate d'isostéaryl-éthylimidonium,
- les sels d'ammonium tels que le chlorure de N,N,N-triméthyl-1-docosanaminium (chlorure de Behentrimonium).

Les compositions selon l'invention peuvent également contenir un ou plusieurs tensioactifs amphotériques comme les N-acyl-aminoacides, tels que les N-alkyl-aminoacétates et le cocoamphodiacétate disodique et les oxydes d'amines tels que l'oxyde de stéaramine ou encore des tensioactifs siliconés comme les diméthicone polyols phosphates tels que celui vendu sous la dénomation « PECOSIL PS 100 » par la société PHOENIX CHEMICAL.

Les agents tensioactifs non ioniques et ioniques de HLB supérieur ou égal à 6 à 25 °C décrits ci-dessus peuvent être également présents en association.

Par ailleurs, les compositions cosmétiques selon la présente invention comprennent de préférence un ou des agent(s) tensioactif(s) de HLB supérieur ou égal à 8 à 25 °C, en particulier de HLB supérieur ou égal à 10, et notamment supérieur ou égal à 12.

Conviennent tout particulièrement à l'invention, à titre d'agents tensioactifs non ioniques de HLB supérieur ou égal à 6 à 25 °C, les esters d'acide gras et des éthers de sorbitol oxyéthylénés et/ou oxypropylénés (pouvant comporter de 1 à 150 groupes oxyéthylénés) comme le polysorbate 20 de HLB 16,7, le polysorbate 40 de HLB 15,6, le polysorbate 60 de HLB 14,9 et le polysorbate 80 de HLB 15,0.

La composition selon l'invention peut comprendre d'autre(s) tensioactif(s) qui sont par exemple introduits dans la composition par l'introduction de la dispersion aqueuse de particules d'un polymère, ces tensioactifs étant ceux classiquement utilisés pour les stabiliser.

### DISPERSION AQUEUSE DE PARTICULES DE POLYMERE/LATEX et PSEUDO-LATEX

Les latex et pseudo-latex sont des dispersions colloïdales de particules de polymère dans une phase liquide aqueuse. Les termes "dispersion aqueuse de particules de polymère" et "latex et pseudo-latex" sont utilisés indifféremment dans le cadre de la description de l'invention.

Les latex sont obtenus généralement par polymérisation ou copolymérisation en suspension ou en émulsion de monomères selon des procédés bien connus de l'homme de l'art. De tels monomères peuvent en particulier être choisis parmi le styrène, le butadiène, l'acrylonitrile, le chloroprène, l'acétate de vinyle, les uréthanes, l'isoprène, l'isobutylène et les acides acrylique ou méthacrylique, maléïque, crotonique, itaconique ou leurs esters ou amides.

Par l'expression "pseudo-latex", on désigne une dispersion constituée de particules généralement sphériques d'un polymère, celles-ci étant obtenues par dispersion du polymère dans une phase aqueuse appropriée.

L'expression "pseudo-latex" ne doit pas être confondue avec l'expression "latex" ou "latex synthétique" qui est également une dispersion constituée de particules d'un polymère qui sont obtenues directement par polymérisation d'un ou plusieurs monomères dans une phase aqueuse appropriée ainsi qu'évoqué ci-dessus.

Ces latex ou pseudo-latex possèdent des propriétés filmogènes avantageuses pour conférer la tenue à l'eau aux compositions cosmétiques objets de l'invention. Les polymères compris dans ces latex ou pseudo-latex sont ainsi aussi nommés polymères filmogènes.

Plus précisément, dans la présente invention, on entend par «polymère filmogène », un polymère apte à former à lui seul ou en présence d'un agent auxiliaire de filmification, un film macroscopiquement continu et adhérent sur les matières kératiniques, et de préférence un film cohésif, et mieux encore un film dont la cohésion et les propriétés mécaniques sont telles que ledit film peut être isolable et manipulable isolément, par exemple lorsque ledit film est réalisé par coulage sur une surface antiadhérente comme une surface téflonnée ou siliconnée.

Parmi les polymères filmogènes pouvant être compris dans le latex ou le pseudo-latex compris dans la composition selon la présente invention, on peut citer les polymères synthétiques du type polycondensat ou du type radicalaire.

Ces polymères filmogènes sont de préférence distincts du polyélectrolyte précédemment défini.

### Polymères synthétiques

Parmi les polycondensats, on peut également citer les polyuréthanes anioniques, cationiques, non ioniques ou amphotères, les polyuréthane-acryliques, les polyuréthanes-polyvinylpyrrolidones, les polyester-polyuréthanes, les polyéther-polyuréthanes, les polyurées, les polyurée/polyuréthanes, et des mélanges de ceux-ci.

Les polyuréthanes peuvent être par exemple un copolymère de polyuréthane aliphatique, cycloaliphatique ou aromatique, de polyurée/polyuréthane ou de polyurée comprenant, seul ou en tant que mélange :
- au moins une séquence d'origine polyester linéaire ou ramifiée, aliphatique et/ou cycloaliphatique et/ou aromatique, et/ou
- au moins une séquence d'origine polyéther aliphatique et/ou cycloaliphatique et/ou aromatique, et/ou
- au moins une séquence siliconée, substituée ou non substituée, ramifiée ou non ramifiée, par exemple de polydiméthylsiloxane ou de polyméthylphénylsiloxane, et/ou
- au moins une séquence comprenant des groupements fluorés.

Les polyuréthanes tels que définis dans l'invention peuvent également être obtenus à partir de polyesters ramifiés ou non ramifiés ou à partir d'alkydes comprenant des hydrogènes mobiles qui sont modifiés au moyen d'une polyaddition avec un diisocyanate et un composé co-réactif bifonctionnel organique (par exemple dihydro, diamino ou hydroxy-amino), comprenant en outre soit un groupement carboxylate ou acide carboxylique, soit un groupement sulfonate ou acide sulfonique, voire un groupement amine tertiaire neutralisable ou un groupement ammonium quaternaire.

On peut également citer les polyesters, les polyesteramides, les polyesters à chaîne grasse, les polyamides et les résines d'époxyester.

Les polyesters peuvent être obtenus de manière connue au moyen de la polycondensation de diacides aliphatiques ou aromatiques avec des diols aliphatiques ou aromatiques ou avec des polyols. L'acide succinique, l'acide glutarique, l'acide adipique, l'acide pimélique, l'acide subérique ou l'acide sébacique peuvent être utilisés comme diacides aliphatiques. L'acide téréphtalique ou l'acide isophtalique, voire un dérivé tel que l'anhydride phtalique, peuvent être utilisés comme diacides aromatiques. L'éthylène glycol, le propylène glycol, le diéthylène glycol, le néopentyl glycol, le cyclohexanediméthanol et le 4,4-N-(1-méthylpropylidène)bisphénol, peuvent être utilisés comme diols aliphatiques. Le glycérol, le pentaérythritol, le sorbitol et le triméthylolpropane peuvent être utilisés comme polyols.

Les polyesteramides peuvent être obtenus de manière analogue aux polyesters, au moyen de la polycondensation de diacides avec des diamines ou des aminoalcools. L'éthylènediamine, l'hexaméthylènediamine, et la méta- ou para-phénylènediamine peuvent être utilisées comme diamine. La monoéthanolamine peut être utilisée comme aminoalcool.

Comme monomère portant un groupement anionique pouvant être utilisé pendant la polycondensation, on peut citer par exemple, l'acide diméthylolpropionique, l'acide trimellitique ou un dérivé tel que l'anhydride trimellitique, le sel de sodium de l'acide 3-sulfopentanediol et le sel de sodium de l'acide 5-sulfo-1,3-benzènedicarboxylique. Les polyesters ayant une chaîne grasse peuvent être obtenus par l'intermédiaire de l'utilisation de diols ayant une chaîne grasse lors de la polycondensation. Les résines d'époxyester peuvent être obtenues par la polycondensation d'acides gras avec un condensat au niveau des extrémités α,ω-diépoxy.

Les polymères radicalaires peuvent être en particulier les polymères ou les copolymères acryliques et/ou vinyliques.

Les polymères acryliques peuvent résulter de la copolymérisation de monomères choisis parmi les esters et/ou les amides de l'acide acrylique ou de l'acide méthacrylique. Comme exemples de monomères du type ester, on peut citer le méthacrylate de méthyle, le méthacrylate d'éthyle, le méthacrylate de butyle, le méthacrylate d'isobutyle, le méthacrylate de 2-éthylhexyle et le méthacrylate de lauryle. Comme exemples de monomères du type amide, on peut citer le N-t-butylacrylamide et le N-t-octylacrylamide.

On utilise de préférence les polymères acryliques obtenus par la copolymérisation de monomères à insaturation éthylénique contenant des groupements hydrophiles, préférablement de nature non ionique, tels que l'acrylate d'hydroxyéthyle, l'acrylate de 2-hydroxypropyle, le méthacrylate d'hydroxyéthyle et le méthacrylate de 2-hydroxypropyle.

Les polymères vinyliques peuvent résulter de l'homopolymérisation ou de la copolymérisation de monomères choisis parmi les esters vinyliques, le styrène ou le butadiène. Comme exemples d'esters vinyliques, on peut citer l'acétate de vinyle, le néodécanoate de vinyle, le pivalate de vinyle, le benzoate de vinyle et le t-butylbenzoate de vinyle.

On peut également utiliser des copolymères d'acrylique/silicone, voire des copolymères de nitrocellulose/ acrylique.

On peut également citer les polymères résultant de la polymérisation radicalaire d'un ou plusieurs monomères radicalaires, à l'intérieur et/ou partiellement à la surface de particules préexistantes d'au moins un polymère choisi parmi le groupe constitué par les polyuréthanes, les polyurées, les polyesters, les polyesteramides et/ou les alkydes. Ces polymères sont généralement appelés « polymères hybrides ».

Comme dispersion aqueuse de polymère filmogène, on peut utiliser les dispersions acryliques vendues sous les dénominations « Neocryl XK-90^{®} », « Neocryl A-1070^{®} », « Neocryl A-1090^{®} », « Neocryl BT-62^{®} », « Neocryl A-1079^{®} » et « Neocryl A-523^{®} » par la société AVECIA-NEORESINS, « Dow Latex 432^{®} » par la société DOW CHEMICAL, « Daitosol 5000 AD^{®} » ou « Daitosol 5000 SJ^{®} » par la société DAITO KASEY KOGYO; « Syntran 5760^{®} » par la société Interpolymer, Allianz Opt^{®} par la société Rohm and Haas, ou encore les dispersions aqueuses de polyuréthane vendues sous les dénominations « Neorez R-981^{®} » et « Neorez R-974^{®} » par la société AVECIA-NEORESINS, les « Avalure UR-405^{®} », « Avalure UR-410^{®} », « Avalure UR-425^{®} », « Avalure UR-450^{®} », « Sancure 875^{®} », « Sancure 861^{®} », « Sancure 878^{®} » et « Sancure 2060^{®} » par la société GOODRICH, « Impranil 85^{®} » par la société BAYER, « Aquamere H-1511^{®} » par la société HYDROMER ; les sulfopolyesters vendus sous le nom de marque « Eastman AQ^{®} » par la société Eastman Chemical Products, les dispersions vinyliques comme le « Mexomère PAM », les dispersions aqueuses de polyvinyl acétate comme le « Vinybran^{®} » de la société Nisshin Chemical ou celles commercialisées par la société UNION CARBIDE, les dispersions aqueuses de terpolymère vinyl pyrrolidone, diméthylaminopropyl méthacrylamide et chlorure de lauryldiméthylpropylmethacrylamidoammonium telles que le Styleze W-d'ISP, les dispersion aqueuse de polymère hybrides polyuréthane/polyacryliques telles que celles commercialisées sous les références « Hybridur^{®} » par la société AIR PRODUCTS ou « Duromer^{®} » de NATIONAL STARCH, les dispersions type core/shell : par exemple celles commercialisées par la société ATOFINA sous la référence Kynar ( core : fluoré - shell : acrylique) ou encore ceux décrits dans le document US 5 188 899 (core : silice - shell : silicone) et leurs mélanges.

La teneur en matière sèche de la dispersion aqueuse de particules d'un polymère est supérieure ou égale à 5 % en poids par rapport au poids total de la composition, en particulier supérieure ou égale à 7 %, en particulier à 10 %, et notamment inférieure à 50 %, et plus particulièrement à 30 %.

Selon certains modes de réalisation de l'invention, le polymère constituant les particules de la dispersion aqueuse est distinct d'un copolymère obtenu par condensation de di-éthylèneglycol, de cyclohexane di-méthanol, d'acide isophtalique et d'acide sulfoisophtalique . Ce copolymère particulier obtenu par condensation de di-éthylèneglycol, de cyclohexane di-méthanol, d'acide isophtalique et d'acide sulfoisophtalique rentre dans la classe des sulfopolyesters. Il est notamment vendu sous le nom de marque "Eastman AQ^{®}" par la société Eastman Chemical Products, par exemple sous les références "EASTMAN AQ 55-S^{®}" ou "EASTMAN AQ 38-S^{®}".

Le polymère constituant les particules de la dispersion aqueuse peut ne pas comporter de monomère ionisable. Les sulfopolyesters cités ci-dessus comportent notamment de tels monomères ionisables, ce qui leur confère une certaine affinité pour l'eau. On préfère au contraire les compositions dans lesquelles les polymères filmogènes ne présentent pas une telle affinité pour l'eau.

Selon un mode de réalisation de l'invention, la composition peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

### EAU ET/OU SOLVANT HYDROSOLUBLE

Les compositions selon l'invention comprennent une phase aqueuse comprenant de l'eau et/ou au moins un solvant hydrosoluble.

Par "solvant hydrosoluble", on désigne dans la présente invention un composé liquide à température ambiante et miscible à l'eau (miscibilité dans l'eau supérieure à 50 % en poids à 25 °C et pression atmosphérique).

Les solvants hydrosolubles utilisables dans les compositions selon l'invention peuvent en outre être volatils.

Parmi les solvants hydrosolubles pouvant être utilisés dans les compositions selon l'invention, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol, les cétones en C₃ et C₄ et les aldéhydes en C₂-C₄.

La phase aqueuse (eau et/ou solvant(s) hydrosoluble(s)) peut être introduite en tant que telle dans la formulation selon l'invention ou y être incorporée par le biais, d'un ou plusieurs ingrédients constituant ladite composition. Ainsi de l'eau peut être notamment introduite dans la composition par le biais de l'introduction du latex ou du pseudo-latex.

La teneur en eau et/ou en solvant(s) hydrosoluble(s) dans les compositions selon l'invention est de préférence comprise entre 20 et 90 % en poids, de façon encore plus préférée entre 25 et 70 % en poids, et plus particulièrement entre 30 et 65 % en poids par rapport au poids total de la composition.

Les compositions cosmétiques peuvent en outre comprendre un sel dissous. Ce sel peut être tout sel inorganique ou organique cosmétiquement acceptable. A ce titre, on peut notamment citer l'EDTA, le déhydroacétate de sodium, NaCl ; KCI, CuSO₄, MgCL₂, NaOH et Na₂SO₄. De préférence, ce sel présente une masse molaire inférieure ou égale à 5000 g/mol, notamment à 800 g/mol. Le sel au sens de la présente invention est de préférence distinct de l'agent tensioactif tel que défini ci-dessus.

Selon une variante particulièrement préférée de l'invention, le latex ou pseudo-latex peut être présent dans une teneur supérieure ou égale à 5 % en poids, et plus particulièrement supérieure ou égale à 10 % en poids par rapport au poids total de la composition en quel cas la composition comprend au moins un sel.

### AGENT STRUCTURANT

Les compositions selon l'invention peuvent comprendre au moins un agent structurant de la phase huileuse ou solvant organique choisi parmi les cires, les polymères semi-cristallins, les gélifiants lipophiles et leurs mélanges.

L'agent structurant peut représenter de 0,1 à 80 % en poids par rapport au poids total de la composition, de préférence de 0,5 à 50 % et de façon encore plus préférée de 1 à 40 % en poids. La quantité en structurant huileux peut être ajustée par l'homme du métier en fonction du propriétés de structuration desdits agents.

### Cire(s)

La cire est d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.

En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

En particulier, les cires convenant à l'invention peuvent présenter un point de fusion supérieur ou égal à 45 °C, et en particulier supérieur ou égal à 55°C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :
Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides, à température ambiante d'origine animale, végétale, minérale ou de synthèse et leurs mélanges.

Les cires pouvant être utilisées dans les compositions selon l'invention présentent généralement une dureté allant de 0,01 MPa à 15 MPa, notamment supérieure à 0,05 MPa et en particulier supérieure à 0,1 MPa.

La dureté est déterminée par la mesure de la force en compression mesurée à 20 °C à l'aide du texturomètre vendu sous la dénomination TA-TX2i^{®} par la société RHEO, équipé d'un mobile en inox en forme de cylindre d'un diamètre de 2 mm en mesurant l'évolution de la force (force de compression ou force d'étirement) (F) en fonction du temps, pendant l'opération suivante :
Le mobile est déplacé à la vitesse de 0,1 mm/s puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,1 mm/s. Pendant le temps de relaxation, la force (force de compression) décroît fortement jusqu'à devenir nulle puis lors du retrait du mobile, la force (force d'étirement) devient négative pour ensuite croître à nouveau vers la valeur 0. La dureté correspond à la force de compression maximale mesurée entre la surface du mobile et la cire au moment de leur mise en contact. La valeur de cette force est exprimée en MPa.

Pour effectuer la mesure de la dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté.

A titre illustratif des cires convenant à l'invention, on peut notamment citer les cires hydrocarbonées comme la cire d'abeilles, la cire de lanoline, les cires d'insectes de Chine, la cire de sumac, les paraffines, certaines cires de polyéthylène et les copolymères cireux ainsi que leurs esters.

On peut aussi citer des cires obtenues par hydrogénation catalytique d'huiles animales ou végétales ayant des chaînes grasses, linéaires ou ramifiées, en C₈-C₃₂, Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale Iso-Jojoba-50^{®}, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S^{®} par la société HETERENE.

On peut encore citer les cires de silicone, les cires fluorées.

On peut également utiliser les cires obtenues par hydrogénation d'huile de ricin estérifiée avec l'alcool cétylique vendues sous les dénominations de Phytowax ricin 16L64^{®} et 22L73^{®} par la société SOPHIM. De telles cires sont décrites dans la demande FR-A- 2792190.

Selon un mode de réalisation particulier, les compositions selon l'invention peuvent comprendre au moins une cire dite cire collante c'est-à-dire possédant un collant supérieur ou égal à 0,7 N.s et une dureté inférieure ou égale à 3,5 MPa.

L'utilisation d'une cire collante peut notamment permettre l'obtention d'une composition cosmétique qui s'applique facilement sur les fibres kératiniques, ayant une bonne accroche sur les fibres kératiniques et qui conduit à la formation d'un maquillage lisse, homogène et épaississant.

La cire collante utilisée peut posséder notamment un collant allant de 0,7 N.s à 30 N.s, en particulier supérieur ou égal à 1 N.s, notamment allant de 1 N.s à 20 N.s, en particulier supérieur ou égal à 2 N.s, notamment allant de 2 N.s à 10 N.s, et en particulier allant de 2 N.s à 5 N.s.

Le collant de la cire est déterminé par la mesure de l'évolution de la force (force de compression ou force d'étirement) en fonction du temps, à 20 °C à l'aide du texturomètre vendu sous la dénomination TA-TX2i^{®} par la société RHEO, équipé d'un mobile en polymère acrylique en forme de cône formant un angle de 45°.

Le protocole de mesure est le suivant :
La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.

Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.

La dureté est mesurée selon le protocole décrit précédemment.

Comme cire collante, on peut utiliser un (hydroxystéaryloxy)stéarate d'alkyle en C₂₀-C₄₀ (le groupe alkyle comprenant de 20 à 40 atomes de carbone), seul ou en mélange, en particulier un 12-(12'-hydroxystéaryloxy)stéarate d'alkyle en C₂₀-C₄₀, de formule (I) : 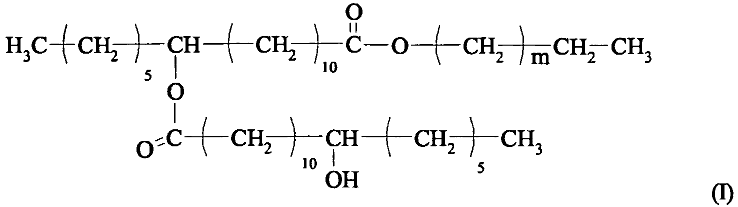 dans laquelle m est un entier allant de 18 à 38, ou un mélange de composés de formule (I).

Une telle cire est notamment vendue sous les dénominations Kester Wax K 82 P^{®} et Kester Wax K 80 P^{®} par la société KOSTER KEUNEN.

Les cires citées ci-dessus présentent généralement un point de fusion commençante inférieur à 45 °C.

Dans la présente invention, on peut également utiliser des cires fournies sous forme de petites particules ayant une dimension exprimée en diamètre « effectif » moyen en volume D[4,3] de l'ordre de 0,5 à 30 micromètres, en particulier de 1 à 20 micromètres, et plus particulièrement de 5 à 10 micromètres, désignées par la suite par l'expression « micro cires ». A des fins de distinction, les cires mises en oeuvre selon l'invention sous forme de fragments de dimension plus élevée sont par la suite désignées par l'expression « cires de type traditionnel ».

Les tailles de particules peuvent être mesurées par différentes techniques, on peut citer en particulier les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer un diamètre de particules et pour certaines d'entre elles une distribution granulométrique.

De préférence, les tailles et les distributions de tailles des particules des compositions selon l'invention, sont mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.

Comme micro cires pouvant être utilisées dans les compositions selon l'invention, on peut citer notamment les micro cires de carnauba telles que celle commercialisée sous la dénomination de MicroCare 350^{®} par la société MICRO POWDERS, les micro cires de cire synthétique telles que celle commercialisée sous la dénomination de MicroEase 114S^{®} par la société MICRO POWDERS, les micro cires constituées d'un mélange de cire de carnauba et de cire de polyéthylène telles que celles commercialisées sous les dénominations de Micro Care 300^{®} et 310^{®} par la société MICRO POWDERS, les micro cires constituées d'un mélange de cire de carnauba et de cire synthétique telles que celle commercialisée sous la dénomination Micro Care 325^{®} par la société MICRO POWDERS, les micro cires de polyéthylène telles que celles commercialisées sous les dénominations de Micropoly 200^{®}, 220^{®}, 220L^{®} et 250S^{®} par la société MICRO POWDERS et les micro cires de polytétrafluoroéthylène telles que celles commercialisées sous les dénominations de Microslip 519^{®} et 519 L^{®} par la société MICRO POWDERS.

Dans les compositions selon l'invention, on peut bien entendu utiliser un mélange de cires et notamment utiliser une ou plusieurs cires de type traditionnel telles que notamment une cire collante et/ou une cire à point de fusion commençante supérieur ou égal à 45 °C, et une ou plusieurs cires dites micro cires. La composition selon l'invention peut comprendre une teneur en cires allant de 0 à 70 % en poids par rapport au poids total de la composition, en particulier elle peut en contenir de 0,5 à 50 %, plus particulièrement de 1 à 30 %.

### Polymères semi-cristallins

On entend par polymère des composés comportant au moins deux motifs, de préférence au moins 3 motifs et plus spécialement au moins 10 motifs de répétition. Par "polymère semi-cristallin", on entend des polymères comportant une partie cristallisable, une chaîne pendante cristallisable ou une séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tribloc ou multibloc, comportant au moins une séquence cristallisable et au moins une séquence amorphe. Par "séquence", on entend généralement au moins 5 motifs de répétition identiques. La ou les séquences cristallisables sont alors de nature chimique différente de la ou des séquences amorphes.

Le polymère semi-cristallin a une température de fusion supérieure ou égale à 30°C (notamment allant de 30°C à 80°C), de préférence allant de 30°C à 60°C. Cette température de fusion est une température de changement d'état du premier ordre.

Cette température de fusion peut être mesurée par toute méthode connue et en particulier à l'aide d'un calorimètre à balayage différentiel (D.S.C).

De façon avantageuse, le ou les polymères semi-cristallins présentent une masse moléculaire moyenne en nombre supérieure ou égale à 1 000, notamment une masse moléculaire moyenne en nombre Mn allant de 2 000 à 800 000, de préférence de 3 000 à 500 000, mieux de 4 000 à 150 000, notamment inférieure à 100 000, et mieux de 4 000 à 99 000. Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitifs du polymère. Avantageusement, la "chaîne pendante cristallisable" peut être une chaîne comportant au moins 6 atomes de carbone.

Le polymère semi-cristallin peut être choisi parmi les copolymères séquencés comportant au moins une séquence cristallisable et au moins une séquence amorphe, les homopolymères et les copolymères portant au moins une chaîne latérale cristallisable par motif de répétition, leurs mélanges.

De tels polymères sont décrits par exemple dans le document EP 1396259.

### A. Polymères semi-cristallins à chaînes latérales cristallisables

On peut citer en particulier ceux définis dans le document US-A-5,156,911 et WO-A-01/19333. Ce sont des homopolymères ou copolymères comportant de 50 à 100 % en poids de motifs résultant de la polymérisation d'un ou plusieurs monomères porteurs de chaîne latérale hydrophobe cristallisable.

Ces homo- ou co-polymères sont de toute nature du moment qu'ils présentent les conditions indiquées précédemment.

### B. Les polymères portant dans le squelette au moins une séquence cristallisable

Ces polymères sont notamment des copolymères séquencés constitués d'au moins 2 séquences de nature chimique différente dont l'une est cristallisable.
- On peut utiliser les polymères séquencés définis dans le brevet US-A-5,156,911 ;
- Les copolymères séquencés d'oléfine ou de cyclooléfine à chaîne cristallisable comme ceux issus de la polymérisation séquencée de :
- cyclobutène, cyclohexène, cyclooctène, norbornène (c'est-à-dire bicyclo(2,2,1)heptène-2), 5-méthylnorbomène, 5-éthylnorbornène, 5,6-diméthylnorbomène, 5,5,6-triméthyl norbornène, 5-éthylidène-norbornène, 5-phényl-norbonène, 5-benzylnorbornène, 5-vinyl norbornène, 1,4,5,8-diméthano-1,2,3,4,4a,5,8a-octahydronaphtalène, dicyclopentadiène ou leurs mélanges,
- avec l'éthylène, le propylène, le 1-butène, le 3-méthyl-1-butène, le 1-hexène, le 4-méthyl-1-pentène, le 1-octène, le 1-décène, le 1-éicosène ou leurs mélanges,
- et en particulier les copoly(éthylène/norbornène) blocs et les terpolymères (éthylène/propylène/éthylidène-norbornène) blocs. On peut aussi utiliser ceux résultants de la copolymérisation séquencée d'au moins 2 α-oléfines en C₂-C₁₆ et mieux en C₂-C₁₂ et encore mieux en C₄-C₁₂ tels que ceux cités précédemment et en particulier les bipolymères séquencés d'éthylène et d'1-octène.
- Les copolymères peuvent être des copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :
   - Séquence cristallisable par nature : a) polyester comme les poly(alkylène téréphtalate), b) polyoléfine comme les polyéthylènes ou polypropylènes.
   - Séquence amorphe et lipophile comme les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe distinctes, on peut citer :
α) les copolymères séquencés poly(ε-caprolactone)-b-poly(butadiène), utilisés de préférence hydrogénés, tels que ceux décrits dans l'article "Melting behavior of poly(ε-caprolactone)-block-polybutadiène copolymers de S. Nojima, Macromolécules, 32, 3727-3734 (1999).
β) les copolymères séquencés poly(butylènetéréphtalate)-b-poly(isoprène) hydrogénés séquencés ou multiséquencés, cités dans l'article "Study of morphological and mechanical properties of PP/PBT de B. Boutevin et al., Polymer Bulletin, 34, 117-123 (1995).
γ) les copolymères séquencés poly(éthylène)-b-copoly(éthylène/propylène) cités dans les articles "Morphology of semi-crystalline block copolymers of ethylene-(ethylene-alt-propylene) de P. Rangarajan et al., Macromdecules, 26, 4640-4645 (1993) et "Polymer agregates with crystalline cores : the system poly(ethylene)-poly(ethylene-propylene) de P. Richter et al., Macromdécules, 30, 1053-1068 (1997).
δ) les copolymères séquencés poly(éthylène)-b-poly(éthyléthylène) cités dans l'article général "Cristallization in block copolymers de I.W. Hamley, Advances in Polymer Science, vol 148, 113-137 (1999).

De préférence, les polymères semi-cristallins convenant aux compositions selon l'invention sont non réticulés.

Ce polymère peut être en particulier choisi parmi les copolymères résultant de la polymérisation d'au moins un monomère à chaîne cristallisable choisi parmi les (méth)acrylates d'alkyle saturés en C₁₄ à C₂₄, les (méth)acrylates de perfluoroalkyle en C₁₁ à C₁₅, les N alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alphaoléfines en C₁₄ à C₂₄, les para-alkyl styrènes avec un groupe alkyle comportant de 12 à 24 atomes de carbone, avec au moins un ester ou amide d'acide monocarboxylique en C₁ à C₁₀ éventuellement fluoré, qui peut être représenté par la formule suivante : 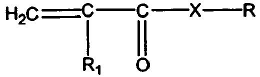 dans laquelle R₁ est H ou CH₃, R représente un groupe alkyle en C₁-C₁₀ éventuellement fluoré et X représente O, NH ou NR₂, où R₂ représente un groupe alkyle en C₁-C₁₀ éventuellement fluoré.

Selon un mode plus particulier de réalisation de l'invention, le polymère est issu d'un monomère à chaîne cristallisable choisi parmi les (méth)acrylates d'alkyle saturés en C₁₄ à C₂₂.

A titre d'exemple particulier de polymère semi-cristallin structurant utilisable dans les compositions selon l'invention, on peut citer les produits Intelimer^{®} de la société Landec décrits dans la brochure "Intelimer^{®} polymers", Landec IP22 (Rev. 4-97). Ces polymères sont sous forme solide à température ambiante (25°C). Ils sont porteurs de chaînes latérales cristallisables et présentent la formule X précédente.

### HUILES

Les compositions selon l'invention peuvent comprendre au moins une huile ou un solvant organique. Il peut s'agir d'un mélange d'huiles ou de solvants organiques.

Les compositions selon l'invention peuvent comprendre une quantité d'huile totale variant de 1 à 30 %, notamment de 8 à 25 % et en particulier de 10 à 20 % en poids par rapport au poids total de la composition, que l'on peut aussi nommer milieu solvant non aqueux.

La ou les huiles présente(s) dans la composition de l'invention peu(ven)t être choisie(s) parmi les huiles volatiles et/ou les huiles non volatiles, et leurs mélanges.

S'avèrent tout particulièrement avantageuses les compositions cosmétiques comprenant essentiellement des huiles volatiles, dans le cadre de la présente invention.

Par « huile ou solvant organique volatile », on entend une huile ou solvant organique (ou milieu non aqueux) susceptible de s'évaporer au contact de la peau en moins d'une heure, à température ambiante et pression atmosphérique.

L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10⁻³ à 300 mm de Hg), et de préférence allant de 1,3 Pa à 8 000 Pa (0,01 à 60 mm de Hg).

Les huiles (ou solvants organiques) volatiles peuvent être des huiles hydrocarbonées, des huiles siliconées, des huiles fluorées ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux "d'Isopars^{®} " ou de "Permetyls^{®}", les esters ramifiés en C₈-C₁₆, le néopentanoate d'iso-hexyle, et leurs mélanges. D'autres huiles hydrocarbonées volatiles comme les distillats de pétrole, notamment ceux vendus sous la dénomination "Shell Solt^{®}" par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 6 centistokes (6.10⁻⁶ m²/s), et ayant notamment de 3 à 6 atomes de silicium, ces silicones comportant éventuellement un ou plusieurs groupes alkyles ou alkoxy ayant de 1 ou 2 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, l'heptaméthyl éthyl trisiloxane, l'heptaméthyl butyl trisiloxane, et leurs mélanges.

On peut également utiliser des solvants organiques volatils notamment fluorés tels que le nonafluorométhoxy butane ou le perfluorométhylcyclopentane.

Avantageusement, le ou les huile(s) volatile(s) est ou sont choisie(s) parmi les huiles volatiles hydrocarbonées ayant de 8 à 16 atomes de carbone, telle que l'isododécane, les huiles volatiles siliconées telles que le décaméthyl cyclopentasiloxane (D5), le dodécaméthyl cyclohexasiloxane (D6) et leurs mélanges.

Les compositions selon l'invention peuvent également comprendre au moins un composé non volatil, non soluble dans l'eau et liquide à température ambiante, notamment au moins une huile ou solvant organique non volatile, qui peut être en particulier choisie parmi les huiles hydrocarbonées et/ou siliconées et/ou fluorées non volatiles.

Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C₄ à C₂₄, ces derniers pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société STEARINERIES DUBOIS ou ceux vendus sous les dénominations de Miglyol 810^{®}, 812^{®} et 818^{®} par la société DYNAMIT NOBEL,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, le benzoate d'alcool en C₁₂ à C₁₅, le laurate d'hexyle, l'adipate de diisopropyle,

l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans la composition de l'invention sont notamment des huiles fluorosiliconées, des polyéthers fluorés, des silicones fluorées telles que décrit dans le document EP-A-847752.

La teneur en huile ou solvant organique non volatile dans les compositions selon l'invention peut varier de 0,01 à 30 % en poids, en particulier de 0,1 à 25 % en poids, et mieux de 0,1 à 20 % par rapport au poids total de la composition.

### ADDITIFS

Bien entendu, l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée.

### POLYMERE FILMOGENE ADDITIONNEL

Les compositions selon l'invention peuvent comprendre outre le polymère filmogène en dispersion dans la phase aqueuse sous forme de particules, requis selon l'invention au moins un polymère filmogène additionnel.

Le polymère filmogène additionnel peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Parmi les polymères filmogènes utilisables dans la composition de la présente invention, on peut citer les polymères hydrosolubles, liposolubles ou encore les polymères en dispersion non aqueuse, détaillés ci-après.

### MATIERE COLORANTE

Les compositions selon l'invention peuvent également comprendre au moins une matière colorante comme les matières pulvérulentes, les colorants liposolubles, les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D C Red 17, le D C Green 6, le -carotène, l'huile de soja, le brun Soudan, le D C Yellow 11, le D C Violet 2, le D C Orange 5, le jaune quinoléine, le rocou.

Ses matières colorantes peuvent être présentes en une teneur allant de 0,01 à 30 % en poids par rapport au poids total de la composition.

### CHARGES

Les compositions selon l'invention peuvent en outre comprendre au moins une charge.

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon^{®} commercialisé sous la dénomination Orgasol^{®} par la société ATOCHEM, de poly-β-alanine et de polyéthylène, les poudres de polymères de tétrafluoroéthylène comme le Téflon^{®}, la lauroyl-lysine, l'amidon, le nitrure de bore, les micro sphères creuses polymériques expansées telles que celles de chlorure de polyvinylidène/acrylonitrile comme celles commercialisées sous la dénomination d'Expancel^{®} par la société NOBEL INDUSTRIE, les poudres acryliques telles que celles commercialisées sous la dénomination Polytrap^{®} par la société DOW CORNING, les particules de polyméthacrylate de méthyle et les microbilles de résine de silicone (Tospearls^{®} de TOSHIBA, par exemple), le carbonate de calcium précipité, le carbonate et l'hydro-carbonate de magnésium, l'hydroxyapatite, les microsphères de silice creuses (Silica Beads^{®} de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium,les particules thermoexpansibles telles que les microsphères non expansées de copolymère de chlorure de vinylidène/d'acrylonitrile/méthacrylate de méthyle ou de copolymère d'homopolymère d'acrylonitrile comme par exemple celles commercialisées respectivement sous les références Expancel^{®} 820 DU 40 et Expancel^{®} 007WU par la Société AK O NOBEL.

Les charges peuvent représenter de 0,1 à 25 %, en particulier de 1 à 20 % en poids par rapport au poids total de la composition.

### FIBRES

Les compositions selon l'invention peuvent en outre comprendre des fibres qui permettent notamment une amélioration de l'effet allongeant lorsque la composition est un mascara.

Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.

Les fibres utilisables dans les compositions de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 m à 10 mm, en particulier de 0,1 mm à 5 mm et plus particulièrement de 0,3 mm à 3,5 mm. Leur section peut être comprise dans un cercle de diamètre allant de 2 nm à 500 m, en particulier allant de 100 nm à 100 m et plus particulièrement de 1 m à 50 m. Le poids ou titre des fibres est souvent donné en denier ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.

Les fibres utilisables dans les compositions de l'invention peuvent être choisies parmi les fibres rigides ou non rigides, elles peuvent être d'origine synthétique ou naturelle, minérales ou organiques.

Par ailleurs, les fibres peuvent être traitées ou non en surface, enrobées ou non, colorées ou non colorées.

A titre de fibres utilisables dans les compositions selon l'invention, on peut citer les fibres non rigides telles que les fibres de polyamide (Nylon^{®}) ou les fibres rigides telles que les fibres de polyimide-amide comme celles vendues sous les dénomination KERMEL^{®}, KERMEL TECH^{®} par la société RHODIA ou de poly-(p-phénylène-téréphtalamide) (ou d'aramide) notamment vendues sous la dénomination Kevlar ® par la société DUPONT DE NEMOURS.

Les fibres peuvent êtres présentes dans une composition selon l'invention en une teneur allant de 0,01 % à 10 % en poids, par rapport au poids total de la composition, en particulier de 0,1 % à 5 % en poids, et plus particulièrement de 0,3 % à 3 % en poids.

### ACTIFS COSMETIQUES

Une composition selon l'invention peut, de plus, comprendre tous les ingrédients classiquement utilisés en cosmétique. Ces ingrédients peuvent notamment être choisis parmi les polymères, notamment les polymères fixants ; les agents conditionneurs de cheveux ; les opacifiants ; les parfums ; les épaississants ; les gélifiants ; les colorants capillaires ; les résines de silicone ; les gomme de silicone ; les conservateurs ; les antioxydants, les actifs cosmétiques ; les filtres solaires ; les agents de stabilisation du pH ; les vitamines ; les hydratants ; les agents antitranspirants ; les agents déodorants ; les composés autobronzants et leurs mélanges. Les quantités de ces différents ingrédients sont celles classiquement utilisées dans les domaines concernés et par exemple de 0,01 à 20 % du poids total de la composition.

### FORMULATION

Une composition selon l'invention peut se présenter sous forme liquide, pâteuse, solide, de mousse ou de spray.

Les compositions selon l'invention peuvent être utilisées pour le maquillage de la peau, des lèvres et/ou des fibres kératiniques d'être humain. Ces compositions trouvent donc une application particulière comme composition de maquillage du corps ou du visage telle que fond de teint, rouge à lèvres, soin pour lèvres, vernis à ongles, soin des ongles, mascara, eye-liner ; composition capillaire telle que composition de coloration des cheveux, composition de protection solaire ; composition à rincer à appliquer avant ou après une coloration, une décoloration, une permanente ou un défrisage ou encore entre les deux étapes d'une permanente ou d'un défrisage ; composition capillaire pour le maintien de la coiffure telle qu'une laque, un gel, une mousse ou un spray de coiffage.

Selon un aspect préféré de l'invention, la composition est sous la forme de rouges à lèvres ou de produits du teint, notamment du type fond de teint, ou encore de mascara.

### PROCEDE

Dans tous les cas, les compositions selon l'invention peuvent être préparées selon des méthodes connues de l'homme du métier.

Dans le cas d'une composition destinée au maquillage des fibres kératiniques, le procédé de préparation des compositions selon l'invention dépend du type de mascara recherché. Il peut dépendre encore en particulier de la nature de la ou des cires éventuellement utilisées.

La présente invention a encore pour objet un procédé de maquillage des fibres kératiniques, dans lequel on applique sur lesdites fibres kératiniques notamment les cils, une composition conforme à l'invention.

Les compositions de l'invention peuvent être en particulier appliquées sur les cils, à l'aide d'une brosse ou d'un peigne.

L'effet épaississant du maquillage, utilisant une composition de l'invention peut par ailleurs être renforcé en sélectionnant tout particulièrement le dispositif pour l'application de ladite composition.

En l'occurrence, il est particulièrement avantageux de procéder, dans le cas du maquillage des cils, à l'application de ladite composition avec une brosse de maquillage telle que décrite dans les brevets FR 2 701 198, FR 2 605 505, EP 792 603 et EP 663 161.

Une composition selon l'invention peut être conditionnée dans un récipient délimitant au moins un compartiment qui comprend ladite composition, ledit récipient étant fermé par un élément de fermeture.

Le récipient est de préférence associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être solidaire du récipient, tel que décrit par exemple le brevet FR 2 761 959. Avantageusement, l'applicateur est solidaire d'une tige qui, elle même, est solidaire de l'élément de fermeture.

L'élément de fermeture peut être couplé au récipient par vissage. Alternativement, le couplage entre l'élément de fermeture et le récipient se fait autrement que par vissage, notamment via un mécanisme à baïonnette, par encliquetage, ou par serrage. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient est de préférence équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

Le contenu des brevets ou demandes de brevets cités précédemment sont incorporés par référence dans la présente demande.

Les exemples qui suivent sont présentés à titre illustratif et non limitatif de l'invention. Sauf indication contraire, les quantités sont données en gramme.

Les tests suivants ont été utilisés pour l'évaluation de la résistance à l'eau et du démaquillage.

### I - Test d'évaluation de la résistance à l'eau et du démaquillage par immersion

### Eprouvette et maquillage

On a réalisé des éprouvettes de faux-cils avec des cheveux caucasiens raides noirs de 19 mm de longueur de frange. On a monté lesdites franges entre deux plaques de 30 mm sur 30 mm.

On a maquillé les cheveux avec la composition testée en effectuant trois fois dix passages espacés de deux minutes à l'aide d'une brosse à mascara.

On a laissé séché pendant une heure à température ambiante (25 °C).

### Descriptif du test

On a immergé l'éprouvette dans l'eau pendant quatre minutes. On a observé visuellement l'apparition d'une dégradation du film (gonflement, irrégularité, décrochement, etc.) et l'on a noté le temps correspondant au début de ce phénomène.

### II - Test d'évaluation du démaquillage sur coton sec

L'éprouvette utilisée pour le test I décrit ci-dessus a ensuite été pincée dans un coton pendant dix secondes puis l'on a tiré le coton pour la démaquiller. On a alors noté l'efficacité du démaquillage en évaluant la quantité de mascara sur le coton et sur les cils.

### Exemple 1 : Mascara

Des formules à haute teneur en latex sont préparées et testées. Les résultats sont rassemblés dans le tableau 1 ci-après.

On constate que les essais 4 et 5, qui sont relatifs à des compositions selon l'invention comprenant à la fois un agent tensioactif non ionique de HLB supérieur à 10 et un polyélectrolyte, présentent des meilleurs résultats au démaquillage que les essais comparatifs 1 à 3.

**Tableau 1**

| | 1 (comparatif) | 2 (comparatif) | 3 (comparatif) | 4 (référence) | 5 (référence) |
|---|---|---|---|---|---|
| Eau | 57,1 | 55,1 | 56,6 | 54,6 | 52,6 |
| SYNTRAN 5760® : Latex acrylique | 30 | 30 | 30 | 30 | 30 |
| HydroxyEthylCellulose | 0.9 | 0.9 | 0.9 | 0.9 | 0.9 |
| Propylène glycol | 5 | 5 | 5 | 5 | 5 |
| Pigment noir | 7 | 7 | 7 | 7 | 7 |
| COSMEDIA SP® | | | 0,5 | 0,5 | 0,5 |
| POLYSORBATE 80 | | 2 | | 2 | 4 |
| Comportement dans l'eau après immersion dans l'eau après 15 minutes (test I) | RAS | RAS | RAS | RAS | RAS |
| Comportement au démaquillage après 1 tiré/pincé sur coton sec après 15 minutes (test II) | Léger transfert sur coton | Léger transfert sur coton | Très léger transfert sur coton | Démaquillage **QUASI-TOTAL** | Démaquillage **TOTAL** |

| | | | | | |
|---|---|---|---|---|---|
| RAS : Rien A Signaler. | | | | | |

| **Exemple 2 :** Mascara | % en poids |
|---|---|
| - Cire de Carnauba (point de fusion : 83-86 °C) | 3,5 |
| - Polyuréthane aliphatique en dispersion aqueuse (Avalure UR 450^{®} de Novéon) | 7,6 MA |
| - Copolymère Acrylamide/AMPS Na dans isohexadécane avec polysorbate 80 ( Simulgel 600^{®} de SEPPIC) | 2,5 |
| - Mono stéarate de glycéryle oxyéthyléné (200 OE) (Simulsol 220TM de SEPPIC) | 4 |
| - Cire d'abeille | 7,4 |
| - Simethicone | 0,1 |
| - Fibres de polyimide-amide (Kermel Tech, 2Dtex, 2 mm de Kermel) | 1 |
| - Disodium EDTA | 0,2 |
| - Oxyde de fer noir | 7 |
| - Butylène glycol | 5 |
| - Ethanol | 3 |
| - Sodium dehydroacetate | 0,2 |
| - Eau | qsp 100 |

| | |
|---|---|
| MA = matière active | |

| **Exemple 3 :** Mascara | % en poids |
|---|---|
| - Cire de Carnauba | 3,5 |
| - Copolymère acrylique et styrène/acrylique en dispersion aqueuse (Syntran 5760) | 8MA |
| - Copolymère acrylamide/AMPS Na dans isohexadécane (Simulgel 600) | 3,5 |
| - Monostéarate de glycéryle oxyéthyléné (200 OE) (Simulsol 200TM) | 4 |
| - Cire d'abeille | 7,4 |
| - Simethicone | 0,1 |
| - Fibres de rayonne | 1 |
| - Disodium EDTA | 0,2 |
| - Oxyde de fer noir | 7 |
| - Butylène glycol | 5 |
| - Ethanol | 3 |
| - Sodium dehydroacetate | 0,2 |
| - Conservateurs | qs |
| - Eau | qsp 100 |

| | |
|---|---|
| MA = matière active | |

## Revendications

1. Composition pour le maquillage et/ou le soin des matières kératiniques, ladite composition ayant une phase continue aqueuse et comprenant au moins une dispersion aqueuse de particules d'un polymère, au moins un polyélectrolyte en une teneur en matière sèche supérieure à 1 % en poids par rapport au poids total de la composition, et au moins un agent tensioactif de HLB supérieur ou égal à 6 en une teneur comprise entre 0,1 % et 10 % en poids par rapport au poids total de la composition, ledit polymère étant présent en une teneur en matière sèche supérieure ou égale à 5 % en poids par rapport au poids total de la composition et ledit polyélectrolyte étant un copolymère acrylamide/acide 2-méthyl 2-[(1-oxo 2-propényl)amino]1-propanesulfonique.

2. Composition cosmétique selon la revendication 1, **caractérisée en ce que** le polyélectrolyte est branché et/ou réticulé.

3. Composition cosmétique selon la revendication 1 ou 2, **caractérisée en ce que** le polyélectrolyte est présent dans la composition cosmétique dans une teneur en matière sèche allant de 1 à 15 % en poids, en particulier de 1,5 à 10 %, et notamment de 2 à 7 % en poids par rapport au poids total de la composition.

4. Composition cosmétique selon l'une quelconque des revendications précédentes, **caractérisée en ce qu'**elle comprend un agent tensioactif dé HLB supérieur ou égal à 10 à 25 °C, et en particulier de HLB supérieur ou égal à 12.

5. Composition cosmétique selon l'une quelconque des revendications précédentes, **caractérisée en ce que** l'agent tensioactif est choisi parmi : les éthers oxyéthylénés et/ou oxypropylénés de glycérol, les éthers oxyéthylénés et/ou oxypropylénés d'alcool gras, les esters d'acide gras et de polyéthylène glycol, les esters d'acide gras et des éthers de glycérol oxyéthylénés et/ou oxypropylénés, les esters d'acide gras et des éthers de sorbitol oxyéthylénés et/ou oxypropylénés, la diméthicone copolyol, la diméthicone copolyol benzoate, les copolymère d'oxyde propylène et d'oxyde d'éthylène, les tensioactifs siliconés, les dérivés d'acides aminés, les sels d'acides gras en C₁₆-C₃₀, les sels d'acides gras polyoxyéthylénés, les esters phosphoriques et leurs sels, les sulfosuccinates, les alkyléthersulfates, les iséthionates, les acylglutamates, les alkyl-imidazolidinium, les sels d'ammonium, et leurs mélanges.

6. Composition cosmétique selon l'une quelconque des revendications précédentes, **caractérisée en ce que** le polymère ne comporte pas de monomère ionisable.

7. Composition cosmétique selon l'une quelconque des revendications précédentes, **caractérisée en ce que** le polymère est obtenu par polymérisation ou copolymérisation de monomères choisis parmi le styrène, le butadiène, l'acrylonitrile, le chloroprène, l'acétate de vinyle, les uréthanes, l'isoprène, l'isobutylène et les acides acrylique ou méthacrylique, maléïque, crotonique, itaconique ou leurs esters ou amides ou par dispersion d'un polymère dans une phase aqueuse.

8. Composition cosmétique selon l'une quelconque des revendications précédentes, **caractérisée en ce que** le polymère est choisi parmi les polymères synthétiques du type polycondensat ou du type radicalaire, et les mélanges de ceux-ci.

9. Composition cosmétique selon la revendication précédente, **caractérisée en ce que** les polymères synthétiques de type polycondensat sont choisis parmi les polyuréthanes anioniques, cationiques, non ioniques ou amphotères, les polyuréthane-acryliques, les polyuréthanes-polyvinylpyrrolidones, les polyester-polyuréthanes, les polyéther-polyuréthanes, les polyurées, les polyurée/polyuréthanes, et des mélanges de ceux-ci, ou encore parmi les polyesters, les polyesteramides, les polyesters à chaîne grasse, les polyamides et les résines d'époxyester.

10. Composition cosmétique selon la revendication précédente, **caractérisée en ce que** les polymères radicalaires sont des polymères ou copolymères acryliques et/ou vinyliques, des copolymères d'acrylique/silicone ou copolymères de nitrocellulose/acrylique ou encore des polymères hybrides.

11. Composition cosmétique selon l'une quelconque des revendications précédentes, **caractérisée en ce que** la teneur en matière sèche de dispersion aqueuse de particules d'un polymère, est supérieure ou égale à 7 %, par rapport au poids total de la composition, en particulier à 10 %, notamment inférieure à 50 %, et en plus particulièrement à 30 %.

12. Composition cosmétique selon l'une quelconque des revendications précédentes, **caractérisée en ce qu'**elle comprend en outre un sel.

13. Composition cosmétique selon la revendication précédente, **caractérisée en ce que** le sel présente une masse molaire inférieure ou égale à 5000 g/mol, notamment à 500 g/mol, et qu'il est choisi parmi l'EDTA, le déhydroacétate de sodium, NaCl, KCl, CuSO₄, MgCL₂, NaOH et Na₂SO₄.

14. Composition cosmétique selon l'une quelconque des revendications précédentes, **caractérisée en ce qu'**elle comprend au moins une huile.

15. Composition selon la revendication précédente, **caractérisée en ce qu'**elle comprend une quantité d'huile variant de 1 à 30 %, notamment de 8 à 25 %, et en particulier mieux de 10 à 20 % en poids par rapport au poids total de la composition.

16. Composition selon l'une des revendications 14 ou 15, **caractérisée en ce que** la ou les huile(s) présente(s) dans la composition peu(ven)t être choisie(s) parmi les huiles volatiles et/ou les huiles non volatiles, et leurs mélanges.

17. Composition selon la revendication précédente, **caractérisée en ce que** les huiles volatiles peuvent être des huiles hydrocarbonées, des huiles siliconées, des huiles fluorées ou leurs mélanges.

18. Composition selon l'une quelconque des revendications précédentes, **caractérisée en ce qu'**elle comprend en outre un agent structurant pouvant être choisi parmi les cires et les polymères semi-cristallins.

19. Composition selon l'une quelconque des revendications précédentes, **caractérisée en ce qu'**elle comprend un polymère filmogène additionnel.

20. Composition selon l'une quelconque des revendications précédentes, **caractérisée en ce qu'**elle contient en outre une matière colorante, des charges et/ou des fibres.

21. Procédé cosmétique de maquillage et/ou de soin des matières kératiniques, **caractérisé en ce qu'**il comprend au moins une étape d'application sur les matières kératiniques, notamment les cils, d'une composition telle que définie selon l'une quelconque des revendications précédentes.

22. Utilisation de l'association d'au moins un polyélectrolyte, ledit polyélectrolyte étant un copolymère acrylamide/acide 2-méthyl 2-[(1-oxo 2-propényl)amino]1-propanesulfonique, et d'au moins un agent tensioactif de HLB supérieur ou égal à 6 à 25 °C, à titre d'additif pour conférer conjointement une bonne tenue à l'eau et une facilité de démaquillage à une composition cosmétique à phase continue aqueuse comprenant au moins une dispersion aqueuse de particules d'un polymère ledit polymère étant présent en une teneur en matière sèche supérieure ou égale à 5 % eh poids par rapport au poids total de la composition, la teneur en matière sèche en polyélectrolyte étant supérieure à 1 % en poids par rapport au poids total de la composition et la teneur en agent tensioactif de HLB supérieur ou égal à 6 étant comprise entre 0,1 % et 10 % en poids par rapport au poids total de la composition.

## Claims

1. Composition for making up and/or caring for keratin materials, the said composition having an aqueous continuous phase and comprising at least one aqueous dispersion of particles of a polymer, at least one polyelectrolyte in a solids content of greater than 1% by weight relative to the total weight of the composition, and at least one surfactant with an HLB of greater than or equal to 6 in a content between 0.1% and 10% by weight relative to the total weight of the composition, the said polymer being present in a solids content of greater than or equal to 5% by weight relative to the total weight of the composition and the said polyelectrolyte being an acrylamide/2-methyl-2[(1-oxo-2 propenyl)amino]-1-propane sulfanic acid copolymer.

2. Cosmetic composition according to Claim 1, **characterized in that** the polyelectrolyte is branched and/or crosslinked.

3. Cosmetic composition according to Claim 1 or 2, **characterized in that** the polyelectrolyte is present in the cosmetic composition in a solids content ranging from 1% to 15% by weight, in particular from 1.5% to 10% and especially from 2% to 7% by weight relative to the total weight of the composition.

4. Cosmetic composition according to any one of the preceding claims, **characterized in that** it comprises a surfactant with an HLB of greater than or equal to 10 at 25°C, in particular an HLB of greater than or equal to 12.

5. Cosmetic composition according to any one of the preceding claims, **characterized in that** the surfactant is chosen from: oxyethylenated and/or oxypropylenated glycerol ethers, oxyethylenated and/or oxypropylenated fatty alcohol ethers, fatty acid esters of polyethylene glycol, oxyethylenated and/or oxypropylenated fatty acid esters of glycerol ethers, oxyethylenated and/or oxypropylenated fatty acid esters of sorbitol ethers, dimethicone copolyol, dimethicone copolyol benzoate, copolymers of propylene oxide and of ethylene oxide, silicone surfactants, amino acid derivatives, C16-C30 fatty acid salts, polyoxyethylenated fatty acid salts, phosphoric esters and salts thereof, sulfosuccinates, alkyl ether sulfates, isethionates, acylglutamates, alkyl-imidazolidiniums and ammonium salts, and mixtures thereof.

6. Comsmetic composition according to any one of the preceding claims, **characterized in that** the polymer does not comprise an ionisable monomer

7. Cosmetic composition according to any one of the preceding claims, **characterized in that** the polymer is obtained by polymerization or copolymerization of monomers chosen from styrene, butadiene, acrylonitrile, chloroprene, vinyl acetate, urethanes, isoprene, isobutylene, and acrylic or methacrylic acid, maleic acid, crotonic acid or itaconic acid or esters or amides thereof, or by dispersion of a polymer in an aqueous phase.

8. Cosmetic composition according to any one of the preceding claims, **characterized in that** the polymer is chosen from synthetic polymers of the polycondensate type or of the free-radical type, and mixtures thereof.

9. Cosmetic composition according to the preceding claim, **characterized in that** the synthetic polymers of polycondensate type are chosen from anionic, cationic, nonionic or amphoteric polyurethanes, polyurethane-acrylics, polyurethane-polyvinylpyrrolidones, polyester-polyurethanes, polyether-polyurethanes, polyureas and polyurea/polyurethanes, and mixtures thereof, or alternatively from polyesters, polyesteramides, fatty-chain polyesters, polyamides and epoxyester resins.

10. Cosmetic composition according to the preceding claim, **characterized in that** the free-radical polymers are acrylic and/or vinyl polymers or copolymers, acrylic/silicone copolymers or nitrocellulose/acrylic copolymers, or alternatively hybrid polymers.

11. Cosmetic composition according to any one of the preceding claims, **characterized in that** the solids content of the aqueous dispersion of particles of a polymer is greater than or equal to 7% and in particular to 10%, and especially less than 50% and more particularly than 30%, relative to the total weight of the composition.

12. Cosmetic composition according to any one of the preceding claims, **characterized in that** it also comprises a salt.

13. Cosmetic composition according to the preceding claim, **characterized in that** the salt has a molar mass of less than or equal to 5000 g/mol and especially 500 g/mol, and **in that** it is chosen from EDTA, sodium dehydroacetate, NaCl, KCl, CuSO4, MgCl2, NaOH and Na2SO4.

14. Cosmetic composition according to any one of the preceding claims, **characterized in that** it comprises at least one oil.

15. Composition according to the preceding claim, **characterized in that** it comprises an amount of oil ranging from 1% to 30%, especially from 8% to 25% and better still in particular from 10% to 20% by weight relative to the total weight of the composition.

16. Composition according to one of Claims 14 or 15, **characterized in that** the oil(s) present in the composition may be chosen from volatile oils and/or non-volatile oils, and mixtures thereof.

17. Composition according to the preceding claim, **characterized in that** the volatile oils may be hydrocarbon-based oils, silicone oils or fluoro oils, or mixtures thereof.

18. Composition according to any one of the preceding claims, **characterized in that** it also comprises a structuring agent that may be chosen from waxes and semi-crystalline polymers.

19. Composition according to any one of the preceding claims, **characterized in that** it comprises an additional film-forming polymer.

20. Composition according to any one of the preceding claims, **characterized in that** it also contains a dyestuff, fillers and/or fibres.

21. Cosmetic process for making up and/or caring for keratin materials, **characterized in that** it includes at least one step of applying to the keratin materials, especially the eyelashes, a composition as defined according to any one the preceding claims.

22. Use of a combination of at least one polyelectrolyte, the said polyelectrolyte being an acrylamide/2-methyl-2-[(1-oxo-2-propenyl)amino]-1-propanesulfonic acid copolymer and of at least one surfactant with an HLB of greater than or equal to 6 at 25°C, as an additive for jointly imparting good water resistance and removability to a cosmetic composition having an aqueous continuous phase comprising at least one aqueous dispersion of particles of a polymer, the said polymer being present in a solids content of greater than or equal to 5% be weight relative to the total weight of the composition, the solids content of the polyelectrolyte being greater than 1% by weight relative to the total weight of the composition and the content of the surfactant with an HLB greater than or equal to 6 being between 0.1% and 10% by weight relative to the total weight of the composition.

## Patentansprüche

1. Zusammensetzung zum Schminken und/oder zur Pflege keratinischer Substanzen, wobei die Zusammensetzung eine kontinuierliche wässrige Phase aufweist und mindestens eine wässrige Dispersion von Teilchen eines Polymers, mindestens einen Polyelektrolyten in einem Trockensubstanzgehalt von größer als 1 Gew.-% bezogen auf das Gesamtgewicht der Zusammensetzung und mindestens ein grenzflächenaktives Mittel mit einem HLB-Wert von größer oder gleich 6 in einem Gehalt im Bereich von 0,1 Gew.-% bis 10 Gew.-%, bezogen auf das Gesamtgewicht der Zusammensetzung, umfasst, wobei das Polymer in einem Trockensubstanzgehalt von größer oder gleich 5 Gew.-% bezogen auf das Gesamtwicht der Zusammensetzung vorhanden ist und der Polyelektrolyt ein Acrylamid/2-Methyl-2-[(1-oxo-2-propenyl)amino]-1-propansulfonsäure-Copolymer ist.

2. Kosmetische Zusammensetzung nach anspruch 1, **dadurch gekennzeichnet, dass** der Polyelektrolyt verzweigt oder vernetzt ist.

3. Kosmetische Zusammensetzung nach Anspruch 1 oder 2, **dadurch gekennzeichnet, dass** der Polyelektrolyt in der kosmetischen Zusammensetzung in einem Trockensubstanzgehalt von 1 bis 15 Gew.-%, insbesondere von 1,5 bis 10 Gew.-%, und speziell von 2 bis 7 Gew.-% bezogen auf das Gesamtgewicht der Zusammensetzung vorhanden ist.

4. Kosmetische Zusammensetzung nach einem der vorhergehenden Ansprüche, **dadurch gekennzeichnet, dass** sie ein grenzflächenaktives Mittel mit einem HLB-Wert von größer oder gleich 10 bei 25 °C, und insbesondere mit einem HLB-Wert von größer oder gleich 12 umfasst

5. Kosmetische Zusammensetzung nach einem der vorhergehenden Ansprüche, **dadurch gekennzeichnet, dass** das grenzflächenaktive Mittel aus folgendem ausgewählt ist: Oyxethylen- und/oder Oxypropylenethern von Glycerin, Oyxethylen- und/oder Oxypropylenethern von Fettalkohol, Estern von Fettsäure und von Polyethylenglycol, Estern von Fettsäure und der Oxyethylen- und/oder Oxypropylenether von Glycerin; Estern von Fettsäure und der Oxyethylen- und/oder Oxypropylenether von Sorbit; Dimethiconcopolyol, Dimethiconcopolyolbenzoat, Copolymeren von Propylen- und Ethylenoxid, Silicon-grenzflächenaktiven Mitteln, Aminosäurederivaten, Salzen von C₁₆-C₃₀-Fettsäuren, Salzen von polyoxyethylenfettsäuren, Phosphorsäureestern und ihren Salzen, Sulfosuccinaten, Alkylethersulfaten, Isethionaten, Acylglutamaten, Alkylimidazolidinium, Ammoniumsalzen, und ihren Gemischen.

6. Kosmetische Zusammensetzung nach einem der vorhergehenden Ansprüche, **dadurch gekennzeichnet dass** das Polymer kein ionisierbares Monomer einschließt.

7. Kosmetische Zusammensetzung nach einem der vorhergehenden Ansprüche, **dadurch gekennzeichnet, dass** das Polymer durch Polymerisation oder Copolymerisation von Monomeren, die ausgewählt sind aus Styrol, Butadien, Acrylnitril, Chloropren, Vinylacetat, Urethanen, Isopren, Isobutylen, und Acryl- oder Methacrylsäure, Maleinsäure, Crotonsäure, Itaconsäure oder ihren Estern oder Amiden oder durch Dispersion von einem Polymer in einer wässrigen Phase erhalten wird.

8. Kosmetische Zusammensetzung nach einem der vorhergehenden Ansprüche, **dadurch gekennzeichnet dass** das Polymer aus synthetischen Polykondensat- oder radikalisch haltenen Polymeren und Gernischen von diesen ausgewählt ist.

9. Kosmetische Zusammensetzung nach dem vorhergehenden Anspruch, **dadurch gekennzeichnet**, die synthetischen Polykondensat-Polymere aus anionischen, kationischen, nichtionischen oder anphoteren Polyurethanen, Polyurethan-Acrylen, Polurethanen-Polyvinylpyrrolidonen, Polyester-Polyurethanen, Polyether-Polyurethanen, Polybarnstoffen, Polyharnstoff/Polyurethanen, und Gemischen von diesen, oder auch aus Polyestern, Polyesteramiden, Polyestern mit Fettsäurekette, Polyamiden und Epoxyesterharzen ausgewählt sind.

10. Kosmetische Zusammensetzung nach dem vorhergehenden Anspruch, **dadurch gekennzeichnet, dass** die radikalisch erhaltenen Polymere acrylische und/oder vinylische Polymere oder Copolymere, Copolymere von Acryl/Silicon oder Copolymere von Nitrocellulose/Acryl oder Hybridpolymere sind.

11. Kosmetische Zusammensetzung nach einem der vorhergehenden Anspräche, **dadurch gekennzeichnet, dass** der Trockensubstanzgehalt der wässrigen Dispersion von Polymerteilchen größer oder gleich 7 % beträgt, bezogen auf das Gesamtgewicht der Zusammensetzung, insbesondere größer oder gleich 10 %, genauer weniger als 50 % und spezieller weniger als 30 % beträgt.

12. Kosmetische Zusammensetzung nach einem der vorhergehenden Ansprüche, **dadurch gekennzeichnet, dass** sie weiterhin ein Salz einschließt.

13. Kosmetische Zusammensetzung nach dem vorhergehenden Anspruch, **dadurch gekennzeichnet, dass** das vorhandene Salz eine Molmasse von kleiner oder gleich 5000 g/mol insbesondere 500 g/mol aufweist und dass es aus EDTA, Natriumdehydroacetat, NaCl, KCl, CuSO₄, MgCl₂, NaOH, Na₂SO₄ ausgewählt ist.

14. Kosmetische Zusammensetzung nach einem der vorhergehenden Ansprüche, **dadurch gekennzeichnet, dass** sie mindestens ein Öl einschließt.

15. Zusammensetzung nach dem vorhergehenden Anspruch, **dadurch gekennzeichnet, dass** sie eine Menge von Öl einschließt, die von 1 bis 30 Gew.-%, insbesondere von 8 bis 25 Gew.-% und spezieller von 10 bis 20 Gew.-%, bezogen auf das Gesamtgewicht der Zusammensetzung, variiert.

16. Zusammensetzung nach einem der Ansprüche 14 oder 15, **dadurch gekennzeichnet, dass** das oder die Öl(e), das (die) in der Zusammensetzung vorhanden ist (sind), aus flüchtigen Ölen und/oder nicht flüchtigen Ölen und ihren Gemischen ausgewählt werdin kann (können).

17. Zusammensetzung nach dem vorhergehenden Anspruch, **dadurch gekennzeichnet, dass** die flüchtigen Öle kohlenwasserstoffhaltigen Öle, silikonhaltige Öle, fluorhaltige Öle oder ihre Gemische sein können.

18. Zusammensetzung nach einem der vorhergehenden Ansprüche, **dadurch gekennzeichnet, dass** sie weiterhin ein Strukturmittel umfasst, das aus Wachsen und semikristallinen Polymeren ausgewählt werden kann.

19. Zusammensetzung nach einem der vorhergehenden Ansprüche, **dadurch gekennzeichnet, dass** sie ein zusätzliches filmbildendes Polymer einschließt.

20. Zusammensetzung nach einem der vorhergehenden Ansprüche, **dadurch gekennzeichnet, dass** sie weiterhin einen Farbstoff, Füllstoffe, und/oder Fasern enthält.

21. Kosmetisches Schmink- und/oder Pflegeverfahren der keratinischen Substanzen, **dadurch gekennzeichnet, dass** es mindestens einen Aufbringschritt einer Zusammensetzung wie in einem der vorhergehenden Ansprüche definiert auf die keratinischen Substanzen, insbesondere auf die Wimpern, umfasst.

22. Verwendung der Kombination von mindestens einem Polyelektrolyten, wobei der Polyclektrolyt ein Acrylamid / 2-Methyl-2-[(1-oxo-2-propenyl)amino]1-propansulfonsäure-Copolymer ist, und von mindestens einem grenzflächenaktiven Mittel mit einem HLB-Wert von größer oder gleich 6 bei 25 °C, als Additiv, um einer kosmetischen Zusammensetzung mit kontinuierlicher wässriger Phase gleichzeitig eine gute Wasserbeständigkeit und Abschmink-Leichtigkeit zu verleihen, umfassend mindestens eine wässrige Dispersion von Teilchen von eine Polymer, wobei das Polymer in einem Trockensubstanzgehalt von größer oder gleich 5 Gew.-%, bezogen auf das Gesamtwicht der Zusammensetzung, vorhanden ist, der Trockensubstanzgehalt an Polyelektrolyt größer als 1 Gew.-% ist, bezogen auf das Gesamtgewicht der Zusammensetzung, und der Gehalt an grenzflächenaktivem Mittel mit einem HLB-Wert von größer oder gleich 6 im Bereich von 0,1 Gew-% bis 10 Gew.-%, bezogen auf das Gesamtgewicht der Zusammensetzung, ist.
